# EUROPÄISCHE PATENTSCHRIFT

(11) **EP 2 016 931 B1**
(45) Veröffentlichungstag und Bekanntmachung des Hinweises auf die Patenterteilung: **29.10.2014**
(21) Anmeldenummer: 08012655.0
(22) Anmeldetag: 14.07.2008
(51) Int. Cl.: A61K 6/027, A61K 6/083

(54) **Dentalkomposite mit niedriger Schrumpfspannung und hoher Biegefestigkeit**
Dental composite with low shrink tensing and high bend stability
Composite dentaire doté d'une tension de réduction moindre et d'une haute résistance à la flexion

(30) Priorität: 20.07.2007 DE 102007034457
(43) Veröffentlichungstag der Anmeldung: 21.01.2009
(60) Teilanmeldung: 10002672.3
(73) Patentinhaber: Heraeus Kulzer GmbH, 63450 Hanau (DE)
(72) Erfinder: Utterodt, Andreas, Dr., 61267 Neu Anspach (DE); Ruppert, Klaus, Dr., 63477 Maintal (DE); Schaub, Matthias, 63589 Linsengericht (DE); Diefenbach, Christine, 65599 Dornburg (DE); Reischl, Kurt, 35799 Merenberg (DE); Hohmann, Alfred, 61389 Schmitten (DE); Eck, Michael, 61389 Schmitten (DE); Schönhof, Nelli, 35619 Braunfels (DE); Schneider, Jutta, 65594 Runkel (DE)
(74) Vertreter: Bendele, Tanja

(56) Entgegenhaltungen:
- DE-A1- 2 816 823
- DE-A1- 3 703 120
- DE-A1-102005 002 845
- DE-A1-102005 021 332

## Beschreibung

Die Erfindung betrifft Dental-Kompositmaterialien mit geringer Schrumpfspannung und hoher Biegefestigkeit.

Lichthärtende Werkstoffe auf Acrylat-/Methacrylatbasis erfahren bei radikalischer Polymerisation aufgrund des sich bei der Polymerisation reduzierenden Molekülabstandes und der damit einhergehenden Dichteerhöhung einen Volumenschrumpf. Dieser kann durch Zugabe von anorganischen Füllstoffen wie z.B. Dentalgläsern oder pyrogenen Kieselsäuren deutlich reduziert werden, da sich ein reduzierter Monomeranteil pro Volumeneinheit ergibt und die Füllstoffe während der Polymerisation nicht schrumpfen.

Bei Dentalanwendungen ist der Volumenschrumpf von großer klinischer Bedeutung, da durch die Materialschrumpfung Zugkräfte auf die Kavitätenwand übertragen werden. Bei Überschreitung einer Maximalkraft kann diese Schrumpfkraft im Extremfall zur Ablösung von der Kavitätenwand führen. In den dadurch entstandenen Randspalt können Bakterien eindringen und in der Folge Sekundärkaries entstehen.

Gemäß DE102005021332A1 wurden bereits lichthärtende Werkstoffe auf Acrylat-/Methacrylatbasis vorgestellt, die eine reduzierte Schrumpfkraft aufweisen. Dies wird durch verschiedene Maßnahmen erreicht: Nicht-agglomerierte Nanofüller, ein Füllstoffgemisch aus grob- und feinteiligen Dentalgläsern, überwiegende Substitution des hochschrumpfenden Verdünners TEDMA durch UDMA (Urethandimethacrylat), Verwendung von Tricyclodecan-Derivaten (im Folgenden abgekürzt TCD) sowie optional die Reduktion der Initiatormenge. Beispielhaft belegt ist dort nur eine Zusammensetzung, und die enthält kein TCD.

Ein Kompositmaterial wird durch innige Vermischung folgender Komponenten hergestellt aus

| | | | |
|---|---|---|---|
| Füllstoffe: | Nicht-agglomerierte Nanopartikel | 6 | Gew. Teile |
| | Dentalglas 1 µm (silanisiert) | 24 | Gew. Teile |
| | Dentalglas 8 µm (silanisiert) | 53 | Gew. Teile |
| Monomere: | Bis-GMA (Bowen) | 11 | Gew. Teile |
| | UDMA | 4 | Gew. Teile |
| | TEDMA | 2 | Gew. Teile |
| Initiator(en): | Campherchinon | 0,1 | Gew. Teile |
| Summe | | 100,1 | Gew. Teile |

Aufgabe der vorliegenden Erfindung ist die Bereitstellung eines Kompositmaterials für Dentalan-wendungen mit geringer Schrumpfkraft und hoher Biegefestigkeit. Insbesondere soll der Quotient Biegefestigkeit/Schrumpfspannung optimiert werden.

Es hat sich gezeigt, dass die in DE102005021332A1 vorgeschlagenen Materialien wesentlich verbessert werden können. Überraschenderweise wurde gefunden, dass das Verhältnis Biegefestigkeit zu Schrumpfspannung gesteigert werden kann, wenn ein Anteil an TCD Monomeren vorhanden ist, von 1-15, besonders bevorzugt über 10 Gew.%.

Die Erfindung betrifft somit

Dental-Kompositmaterialien mit einem Gesamtfüllstoffgehalt von 70 bis 95 Gew.% enthaltend
A) in der Füllstoffkomponente 0,5 bis 10 Gew.% nicht-agglomerierte Nanofüller mit Partikelgrößen von 1 bis 50 nm;
B) in der Füllstoffkomponente mindestens 60 Gew.% eines Füllstoffgemischs aus 50 bis 90 % grob- und 10 bis 50 % feinteiligen Dentalgläsern, welche ein Größenverhältnis, bezogen auf die mittlere Partikelgröße (d₅₀-Wert), von
   feinteilig zu grobteilig von 1:4 bis 1:30 aufweisen,
C) als Monomerkomponente eine Monomermischung aus
   i. 60 - 80 % Bis-GMA und eines Mitglieds der Gruppe TCD-di-HEMA oder TCD-di-HEA
   ii. 10 bis 18 % UDMA
   iii. Rest TEDMA und/oder multifunktionelle Vernetzer
D) bis 1 % Initiator(en) und
E) optional in der Füllstoffkomponente mindestens ein weiteres Dentalglas mit sich von den grob- und feinteiligen Dentalgläsern unterscheidender Partikelgröße.

Nicht agglomerierte Nanofüller sind an sich bekannt und z.B. in WO 0130305 A1 oder am Beispiel von SiO₂ in DE 196 17 931 A1 beschrieben. Sie gehören erfindungsgemäß vorzugsweise der Gruppe bestehend aus: SiO₂, ZrO₂, TiO₂, Al₂O₃ sowie Mischungen aus mindestens zwei dieser Stoffe an.

Sie können - wie in DE 196 17 931 A1 beschrieben - in organischen Lösungsmitteln dispergiert sein, aber auch in Wasser oder Wasser enthaltenden Lösungsmittelmischungen.

Als Dentalgläser eignen sich besonders Bariumglaspulver und/oder Strontiumglaspulver. Die mittlere Partikelgröße der grobteiligen Dentalgläser beträgt vorzugsweise 5-10 µm, insbesondere um 7 µm und die der feinteiligen 0,5 bis 2 µm, insbesondere 1 µm. Optional vorhandene weitere Dentalgläser haben z.B. mittlere Korngrößen von 2-5 oder 10-50 µm.

Die Füllstoffkomponente kann demnach Dentalgläser mit insgesamt drei oder mehr Kornfraktionen aufweisen. Sie kann auch weitere, herkömmliche, auf dem Dentalgebiet übliche Füllstoffe enthalten, wie etwa Quarz-, Glaskeramik- oder Mischungen davon. Darüber hinaus können die Komposite Füllstoffe zur Erzielung einer erhöhten Röntgenopazität enthalten. Die mittlere Partikelgröße des röntgenopaken Füllstoffs liegt vorzugsweise im Bereich von 100 bis 300 nm, insbesondere 180 bis 300 nm. Als röntgenopake Füllstoffe eignen sich z.B. die in der DE 35 02 594 A1 beschriebenen Fluoride der Seltenen Erdmetalle, d. h. die Trifluoride der Elemente 57 bis 71. Ein besonders bevorzugt verwendeter Füllstoff ist Ytterbiumfluorid, insbesondere Ytterbiumtrifluorid mit einer mittleren Partikelgröße von etwa 300 nm. Die Menge des röntgenopaken Füllstoffs beträgt vorzugsweise 10 bis 50 Gew.-%, besonders bevorzugt 20 bis 30 Gew.-%, bezogen auf den Gesamtfüllstoffgehalt.

Außerdem können gefällte Mischoxide, wie beispielsweise ZrO₂/SiO₂, als Füllstoffe eingesetzt werden. Bevorzugt sind Mischoxide mit einer Partikelgröße von 200 bis 300 nm und insbesondere etwa 200 nm. Die Mischoxidpartikel sind vorzugsweise kugelförmig und weisen eine einheitliche Größe auf. Die Mischoxide haben vorzugsweise einen Brechungsindex von 1,52 bis 1,55. Gefällte Mischoxide werden vorzugsweise in Mengen 25 bis 75 Gew. % und besonders 40 bis 75 Gew.% verwendet.

Die Füllstoffe sind zur Verbesserung der Haftung zwischen Füllstoff und organischer Matrix bevorzugt silanisiert. Als Haftvermittler eignet sich besonders alpha - Methacryloxypropyltrimethoxysilan. Die Menge des eingesetzten Haftvermittlers richtet sich nach der Art und BET-Oberfläche des Füllstoffs.

Als multifunktionelle Vernetzer kommen außer TEDMA und UDMA in Frage: Diethylenglycoldi(meth)acrylat, Decandioldi(meth)acrylat, Trimethylolpropantri(meth)acrylat, Pentaerythrittetra(meth)acrylat sowie Butandioldi(meth)acrylat, 1,10-Decandioldi(meth)acrylat, 1,12-Dodecandioldi(meth)acrylat.

Zur Initiierung der Polymerisation enthalten die Komposite einen Polymerisationsinitiator, beispielsweise einen Initiator für die radikalische Polymerisation. Je nach Art des verwendeten Initiators können die Mischungen kalt, durch Licht oder heiß polymerisierbar sein.

Als Initiatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch alpha, alpha '-Azo-bis(isobutyroethylester), Benzpinakol und 2,2'-Dimethylbenzpinakol sind geeignet.

Als Photoinitatoren kommen z.B. Benzoinalkylether oder -ester, Benzilmonoketale, Acylphosphinoxide oder aliphatische und aromatische 1.2-Diketoverbindungen, wie beispielsweise 2,2-Diethoxyacetophenon 9,10-Phenanthrenchinon, Diacetyl, Furil, Anisil, 4,4'-Dichlorbenzil und 4,4'-Dialkoxybenzil oder Campherchinon, in Frage. Photoinitiatoren werden vorzugsweise zusammen mit einem Reduktionsmittel verwendet. Beispiele für Reduktionsmittel sind Amine wie aliphatische oder aromatische tertiäre Amine, beispielsweise N,N-Dimethyl-p-toluidin oder Triethanolamin, Cyanethylmethylanilin, Triethylamin, N,N-Dimethylanilin, N-Methyldiphenylamin, N,N-Dimethyl-sym.-xylidin, N,N-3,5-Tetramethylanilin und 4-Dimethylaminobenzoesäureethylester oder organische Phosphite. Gängige Photoinitiatorsysteme sind z.B. Campherchinon plus Ethyl-4-(N,N-dimethylamino)benzoat, 2-(Ethylhexyl)-4-(N,N-dimethylamino)benzoat oder N,N-Dimethylaminoethylmethacrylat.

Als Initiator für die durch UV-Licht initiierte Polymerisation eignet sich besonders 2,4,6-Trimethylbenzoyldiphenylphosphinoxid. UV-Photoinitiatoren können allein, in Kombination mit einem Initiator für sichtbares Licht, einem Initiator für die Kalthärtung und/oder einem Initiator für die Heißhärtung eingesetzt werden.

Als Initiatoren für die Kaltpolymerisation werden Radikale liefernde Systeme, z. B. Benzoyl- bzw. Lauroylperoxid zusammen mit Aminen wie N,N-Dimethyl-sym.-xylidin oder N,N-Dimethyl-p-toluidin verwendet.

Es können auch dual härtende Systeme verwendet werden, z. B. Photoinitiatoren mit Aminen und Peroxiden.

Die Initiatoren werden vorzugsweise in Mengen von 0,01 bis 1 Gew.% bezogen auf die Gesam-tmasse der Mischung verwendet.

Bei der Kaltpolymerisation kann es zweckmäßig sein, wenn das Kompositmaterial aufgeteilt in zwei Komponenten vorliegt, die zur Aushärtung durch Vermischen vorgesehen sind. Es ist auch möglich, das Material so bereitzustellen, dass es sowohl durch Licht als auch durch Vermischen zweier Komponenten zu härten ist.

Erfndungsgemäße Kompositmaterialien zeigen als Dentalmaterialien bevorzugt einen Quotienten Biegefestigkeit/Schrumpfspannung von >/= 35, bevorzugt >/= 40, besonders bevorzugt >/=50.

Die folgenden Beispiele erläutern die Erfindung näher. Teile- und Prozentangaben beziehen sich wie in der übrigen Beschreibung auf das Gewicht, sofern nicht anders angegeben.

### Beispiele

Die Messergebnisse (Tabelle II) für die in der folgenden Tabelle I aufgeführten Mischungen 312, 349, 357, 363, 307 und 206 (Vergleich, optimiert ohne TCD) zeigen, dass mit steigendem TCD Gehalt der Quotient Biegefestigkeit/Schrumpfspannung ansteigt. TCD Anteile über 10% zeigen Werte über 50.

**Tabelle I Rezepturen**

| Komponenten | Rezeptur **363** | Rezeptur **312** | Rezeptur **357** | Rezeptur **349** | Rezeptur **307** |
|---|---|---|---|---|---|
| | | | | Vergleich | Vergleich |
| UDMA | 4,03 | 4,02 | 4,03 | 2,00 | 3,92 |
| Bis-GMA | 10,76 | 9,15 | 9,15 | | |
| TEGDMA | 1,11 | 1,11 | 1,11 | 2,66 | 0,95 |
| TCDDIHEA | | 1,61 | 1,61 | 10,50 | 12,35 |
| Mutifunktionales Urethanmonomer | 0,50 | 0,50 | 0,50 | 0,50 | 1,18 |
| BHT | 0,04 | 0,04 | 0,04 | 0,04 | 0,04 |
| Nano SiO2 (Dispersion) | 4,00 | 4,00 | 4,00 | 4,00 | 4,8 |
| Bariumaluminosilikatglasfüller 0.85 µ silanisiert | 39,50 | 15,80 | 39,50 | 40,00 | 50,28 |
| Bariumaluminosilikatglasfüller 2 µ silanisiert | | 23,70 | | | 7,66 |
| Bariumaluminosilikatglasfüller 5 µ silanisiert | 39,50 | 39,50 | 39,50 | 40,00 | 18,53 |
| Licht-Stabilisator 1 | 0,09 | 0,09 | 0,09 | 0,05 | 0,07 |
| Licht-Stabilisator 2 | 0,26 | 0,26 | 0,26 | 0,13 | 0,02 |
| DL Campherchinon | 0,03 | 0,03 | 0,03 | 0,02 | 0,02 |
| Coinitiator | 0,14 | 0,14 | 0,14 | 0,07 | 0,17 |
| PPD | 0,02 | 0,02 | 0,02 | 0,01 | 0,01 |
| Pigmente | 0,02 | 0,03 | 0,02 | 0,02 | |
| **Gesamt** | 100 | 100 | 100 | 100 | 100 |

**Tabelle II Messresultate**

| Material | Charge | Elastizitätsmodul in Mpa | | TCD-Monomer | Schrumpfspannung in Mpa¹ | | 3-Punkt Biegefestigkeit in Mpa | | **Quotient** | Std.abw. Quotient |
|---|---|---|---|---|---|---|---|---|---|---|
| | | E-Modul | Std.-abw. | | 24 h MW | Std.-abw. | Biegefestigkeit | Std.-abw. | | |
| Tetric Evo Ceram (Ivoclar Vivadent) | # G 20087 | 9172 | 308 | | 3,603 | 0,158 | 105,0 | 5,6 | **29,14** | 2,0 |
| Premise (Kerr) | # 438811 | 7840 | 239 | | 3,679 | 0,143 | 93,0 | 5,5 | **25,28** | 1,8 |
| InTen-S(Ivoclar Vivadent) | # G 02023 | 9506 | 690 | | 3,822 | 0,181 | 109,0 | 11,9 | **28,52** | 3,4 |
| Filtek Supreme XT (3M Espe) | # 4 AP | 6528 | 374 | | 4,228 | 0,336 | 111,0 | 10,6 | **26,25** | 3,3 |
| EsthetX (Dentsply) | # 305000182 | 11606 | 585 | | 4,388 | 0,240 | 109,0 | 13,6 | **24,84** | 3,4 |
| Herculite XRV (Kerr) | # 06-1262 | 9585 | 305 | | 4,636 | 0,124 | 138,0 | 11,9 | **29,77** | 2,7 |
| Filtek Z250 (3M Espe) | # 4 LPJ | 12174 | 420 | | 4,887 | 0,289 | 153,0 | 15,3 | **31,31** | 3,6 |
| Spectrum TPH (Dentsply) | # 0612001961 | 10367 | 334 | | 4,900 | 0,250 | 127,0 | 10,5 | **25,92** | 2,5 |
| Xtra Fil (VOCO) | # 530835 | 16377 | 669 | | 4,912 | 0,212 | 132,0 | 8,0 | **26,87** | 2,0 |
| Quixfil (Dentsply) | # 0404000401 | 16257 | 783 | | 5,041 | 0,126 | 130,0 | 10,5 | **25,79** | 2,2 |
| Venus (Heraeus) | # 010119 | 9160 | 539 | | 5,180 | 0,252 | 120,0 | 9,6 | **23,17** | 2,2 |
| TPH³ (Dentsply) | # 0409171 | 9804 | 330 | | 5,515 | 0,167 | 128,0 | 13,0 | **23,21** | 2,5 |
| Grandio (VOCO) | # 441238 | 16498 | 544 | | 5,686 | 0,202 | 136,0 | 13,3 | **23,92** | 2,5 |
| | | | | | | | | | | |
| Vergleichsbeispiel 363 | | 13658 | 548 | 0 | 3,845 | 0,193 | 149,0 | 6,2 | **38,75** | 2,5 |
| Beispiel 312 | | 15767 | 560 | 1,61 | 3,250 | 0,087 | 130,0 | 10,4 | **40,00** | 3,4 |
| Beispiel 357 | | 13727 | 272 | 1,61 | 4,060 | 0,185 | 145,0 | 9,4 | **35,71** | 2,8 |
| Beispiel 349 Vergleich | | 14262 | 661 | 10,5 | 3,330 | 0,143 | 171,0 | 7,6 | **51,35** | 3,2 |
| Beispiel 307 Vergleich | | 13836 | 272 | 12,35 | 3,269 | 0,189 | 172,0 | 11,0 | **52,62** | 4,5 |

| | | | | | | | | | | |
|---|---|---|---|---|---|---|---|---|---|---|
| ¹ gemessen nach der Bonded-Disc-Methode - Dental Materials (2004) 20, 88-95) | | | | | | | | | | |

## Patentansprüche

1. Dental-Kompositmaterialien mit einem Gesamtfüllstoffgehalt von 70 bis 95 Gew% enthaltend
A) in der Füllstoffkomponente 0,5 bis 10 Gew.% nicht-agglomerierte Nanofüller mit Partikelgrößen von 1 bis 50 nm;
B) in der Füllstoffkomponente mindestens 60 Gew.% eines Füllstoffgemischs aus 50 bis 90 % grob- und 10 bis 50 % feinteiligen Dentalgläsern, welche ein Größenverhältnis, bezogen auf die mittlere Partikelgröße (d₅₀-Wert), von
feinteilig zu grobteilig von 1:4 bis 1:30 aufweisen,
aufweisen;
C) als Monomerkomponente eine Monomermischung aus
i. 60 - 80 Gew. % Bis-GMA und einem Mitglied der Gruppe TCD-di-HEMA und TCD-di-HEA,
ii. 10 bis 18 Gew. % UDMA ,
iii. Rest TEDMA und/oder multifunktionelle Vernetzer,
D) bis 1 Gew. % Initiator(en) und
E) optional in der Füllstoffkomponente mindestens ein weiteres Dentalglas mit sich von den grob- und feinteiligen Dentalgläsern unterscheidender Partikelgröße,
**dadurch gekennzeichnet, dass** der Anteil an TCD-Monomeren in der Gesamtzusammensetzung 1-15 Gew.% beträgt, und dass der Quotient Biegefestigkeit/Schrumpfspannung >/= 35 beträgt.

2. Dental-Kompositmaterialien nach Anspruch 1, **dadurch gekennzeichnet, dass** der Anteil an TCD-Monomeren in der Gesamtzusammensetzung 10 -15 Gew.% beträgt.

3. Dental-Kompositmaterialien nach Anspruch 1 oder 2, **dadurch gekennzeichnet, dass** der Quotient Biegefestigkeit/Schrumpfspannung >/= 40 beträgt.

4. Dental-Kompositmaterialien nach Anspruch 1 oder 2, **dadurch gekennzeichnet, dass** der Quotient Biegefestigkeit/Schrumpfspannung >/= 50 beträgt.

## Claims

1. Dental composite materials with a total filling agent content of 70 to 95 % by weight, containing
A) in the filling agent component, 0.5 to 10 % by weight non-agglomerated nanofillers of particle sizes of 1 to 50 nm;
B) in the filling agent component, at least 60 % by weight of a filling agent mixture made of 50 to 90 % coarse-particled dental glasses and 10 to 50 % fine-particled dental glasses, whereby the size ratio, relative to the mean particle size (d₅₀ value) of fine particles to coarse particles is 1:4 to 1:30;
C) as monomer component, a monomer mixture made of
i. 60 - 80 % by weight bis-GMA and a member of the group of TCD-di-HEMA and TCD-di-HEA;
ii. 10 to 18 % by weight UDMA;
iii. the remainder being TEDMA and/or multi-functional cross-linkers;
D) up to 1 % by weight initiator(s); and
E) optionally in the filling agent component, at least one further dental glass of a different particle size than the coarse-particled and fine-particles dental glasses,
**characterised in that** the fraction of TCD monomers in the total composition is 1 - 15 % by weight, and **in that** the ratio of flexural strength to shrink tension is >/= 35.

2. Dental composite materials according to claim 1, **characterised in that** the TCD monomers fraction in the total composition is 10 - 15 % by weight.

3. Dental composite materials according to claim 1 or 2, **characterised in that** ratio of flexural strength to shrink tension is >/= 40.

4. Dental composite materials according to claim 1 or 2, **characterised in that** the ratio of flexural strength to shrink tension is >/= 50.

## Revendications

1. Matériaux composites dentaires ayant une teneur en charge totale de 70 à 95 % en poids comprenant
A) 0,5 à 10 % en poids de nanocharges non-agglomérées avec des tailles particulaires de 1 à 50 nm dans le composant de charge ;
B) au moins 60 % en poids d'un mélange de charges constitué de 50 à 90 % de verres dentaires à particules grossières et 10 à 50 % en poids de verres dentaires à particules fines dans le composant de charge, qui présentent une proportion par rapport à la taille particulaire moyenne (valeur d50) entre particules fines et particules grossières de 1:4 à 1:30 ;
C) un mélange de monomères constitué de
i. 60 - 80 % en poids de bis-GMA et d'un membre du groupe TCD-di-HEMA et TCD-di-HEA,
ii. 10 à 18 % en poids de UDMA,
iii. le reste de TEDMA et/ou d'agents de réticulation multifonctionnels en tant que composant monomère,
D) jusqu'à 1 % en poids d'initiateur(s) et
E) en option dans le composant de charge, au moins un verre dentaire supplémentaire ayant une taille particulaire différente des verres dentaires à particules grossières et à particules fines,
**caractérisé en ce que** la teneur en monomères TCD dans la composition totale est de 1 - 15 % en poids et que le rapport résistance à la flexion/tension de retrait est de >/= 35.

2. Matériaux composites dentaires selon la revendication 1, **caractérisé en ce que** la teneur en monomères TCD dans la composition totale est de 10 - 15 % en poids.

3. Matériaux composites dentaires selon la revendication 1 ou 2, **caractérisé en ce que** le rapport résistance à la flexion/tension de retrait est de >/= 40.

4. Matériaux composites dentaires selon la revendication 1 ou 2, **caractérisé en ce que** le rapport résistance à la flexion/tension de retrait est de >/= 50.
